# EUROPEAN PATENT APPLICATION

(11) **EP 4 585 860 A1**
(43) Date of publication of application: **16.07.2025**
(21) Application number: 23877510.0
(22) Date of filing: 12.09.2023
(51) Int. Cl.: F24F 1/0076, F24F 8/22, F24F 11/70, A61L 2/10, A61L 9/20, F24F 120/00, F24F 130/30

(54) **METHOD FOR STERILIZING INDOOR UNIT FAN, AND ELECTRONIC DEVICE THEREFOR**

(30) Priority: 14.10.2022 KR 20220132737
(71) Applicant: Samsung Electronics Co., Ltd., Suwon-si, Gyeonggi-do 16677 (KR)
(72) Inventor: LEE, Hyoshin, Suwon-si, Gyeonggi-do 16677 (KR); GU, Chiwook, Suwon-si, Gyeonggi-do 16677 (KR); SONG, Jungmi, Suwon-si, Gyeonggi-do 16677 (KR); YANG, Jaehyuk, Suwon-si, Gyeonggi-do 16677 (KR); LEE, Jaekil, Suwon-si, Gyeonggi-do 16677 (KR)
(74) Representative: Walaski, Jan Filip
(86) International application number: PCT/KR2023/013665
(87) International publication number: WO 2024/080580

(57) **Abstract**

Provided are an air conditioner and method for sterilizing a fan. According to an embodiment, the air conditioner may include a fan, a fan motor, an ultraviolet (UV) light output module, at least one memory storing one or more instructions, and at least one processor, and the at least one processor may be configured to execute the one or more instructions stored in the memory to sterilize a partial region of the fan by controlling the UV light output module to irradiate UV light onto the partial region, change a region to be sterilized by rotating the fan after sterilizing the partial region, determine, when the fan stops, whether a region of the fan being irradiated with the UV light is a region sterilized by the UV light output module, and based on determining that the region of the fan being irradiated with the UV light is a sterilized region, change a region to be sterilized by driving the fan motor to rotate the fan again.

## Description

### Technical Field

The present disclosure relates to an electronic device for sterilizing an indoor unit fan, a control method of the electronic device, and a computer-readable recording medium having stored therein a computer program for performing the control method of the electronic device.

### Background Art

An air conditioner sucks in indoor air, controls the condition of the air, such as the temperature or humidity of the air, and then discharges the air back into the room. In this case, the air conditioner may filter out dust and other debris in the sucked in air through a filter.

However, as the usage time of the air conditioner increases, dust that is not filtered out by the filter accumulates inside the air conditioner. Mold and bacteria in the air (e.g., Escherichia coli and Staphylococcus aureus) also accumulate inside the air conditioner. In this case, mold and bacteria are absorbed into the dust inside the air conditioner, and the dust becomes food for the mold and bacteria, making it easy for the mold and bacteria to grow. In particular, water remaining in a heat exchanger after cooling is finished or moisture inside the air conditioner creates an ideal environment for mold and bacteria to grow.

### Disclosure of Invention

### Solution to Problem

According to a first aspect of an embodiment of the present disclosure, there may be provided an air conditioner including a fan, a fan motor, an ultraviolet (UV) light output module, at least one memory storing one or more instructions, at least one processor, wherein the at least one processor is configured to execute the one or more instructions stored in the memory to sterilize a partial region of the fan by controlling the UV light output module to irradiate UV light onto the partial region, change a region to be sterilized by rotating the fan after sterilizing the partial region, determine, when the fan stops, whether a region of the fan being irradiated with the UV light is a region sterilized by the UV light output module, and based on determining that the region of the fan being irradiated with the UV light is a sterilized region, change a region to be sterilized by driving the fan motor to rotate the fan again.

According to a second aspect of an embodiment of the present disclosure, there may be provided a method of sterilizing a fan, the method including sterilizing a partial region of the fan by irradiating UV light onto the partial region, changing a region to be sterilized by rotating the fan after sterilizing the partial region, determining, when the fan stops, whether a region of the fan being irradiated with the UV light is a sterilized region, and based on determining that the region of the fan being irradiated with the UV light is a sterilized region, changing a region to be sterilized by rotating the fan again.

According to a third aspect of an embodiment of the present disclosure, there may be provided a computer-readable recording medium having recorded thereon a program for performing the method according to the second aspect on a computer.

### Brief Description of Drawings

FIG. 1 illustrates a method, performed by an air conditioner, of sterilizing an indoor unit fan, according to an embodiment of the present disclosure.
FIG. 2 is a block diagram of an air conditioner according to an embodiment of the present disclosure.
FIG. 3 is a flowchart of a method, performed by an air conditioner, of sterilizing an indoor unit fan, according to an embodiment of the present disclosure.
FIG. 4 is a flowchart of a method, performed by an air conditioner, of determining a location of a partial region of a fan being irradiated with ultraviolet (UV) light based on an intensity of the light reflected by the fan, according to an embodiment of the present disclosure.
FIG. 5 illustrates a method, performed by an air conditioner, of determining a location of a partial region of a fan being irradiated with UV light based on an intensity of the light reflected by the fan, according to an embodiment of the present disclosure.
FIG. 6 illustrates a method, performed by an air conditioner, of determining whether a partial region of a fan being irradiated with UV light is a sterilized region based on an intensity of reflected light, according to an embodiment of the present disclosure.
FIG. 7 illustrates a method, performed by an air conditioner, of determining whether a partial region of a fan being irradiated with UV light is a sterilized region based on a sensor value of Hall sensors, according to an embodiment of the present disclosure.
FIG. 8 illustrates a method, performed by an air conditioner, of changing a region of a fan to be sterilized, according to an embodiment of the present disclosure.
FIG. 9 is a flowchart of a method, performed by an air conditioner, of sterilizing a fan, according to an embodiment of the present disclosure.
FIG. 10 illustrates a method, performed by an air conditioner, of starting sterilization of a fan, according to an embodiment of the present disclosure.
FIG. 11 illustrates a method, performed by an air conditioner, of indicating that sterilization is being performed, according to an embodiment of the present disclosure.
FIG. 12 is a block diagram of an air conditioner according to an embodiment of the present disclosure.

### Mode for the Invention

Throughout the present disclosure, the expression "at least one of a, b, or c" indicates only a, only b, only c, both a and b, both a and c, both b and c, all of a, b, and c, or variations thereof.

An embodiment of the present disclosure will be described more fully hereinafter with reference to the accompanying drawings so that the embodiment may be easily implemented by one of ordinary skill in the art. However, the present disclosure may be implemented in different forms and should not be construed as being limited to embodiments set forth herein. Furthermore, parts not related to descriptions of the present disclosure are omitted to clearly explain the present disclosure in the drawings, and like reference numerals denote like elements throughout.

As the terms used herein, general terms that are currently widely used are selected by taking functions in the present disclosure into account, but the terms may refer to various other terms according to an intention of one of ordinary skill in the art, precedent cases, advent of new technologies, etc. Thus, the terms used herein should be defined not by simple appellations thereof but based on the meaning of the terms together with the overall description of the present disclosure.

Furthermore, although the terms such as "first", "second", etc. may be used herein to describe various elements or components, these elements or components should not be limited by the terms. The terms are only used to distinguish one element or component from another element or component.

In addition, the terms used herein are only used to describe particular embodiments of the present disclosure, and are not intended to limit the present disclosure. Singular expressions used herein are intended to include plural expressions as well unless the context clearly indicates otherwise. Furthermore, throughout the specification, when a part is referred to as being "connected" or "coupled" to another part, it will be understood that the part may be directly connected to or electrically coupled to the other part with one or more intervening elements therebetween. Furthermore, when a part "includes" or "comprises" an element, unless there is a particular description contrary thereto, it is understood that the part may further include other elements, not excluding the other elements.

Expressions such as "in some embodiments" or "in an embodiment" described in various parts of this specification do not necessarily refer to the same embodiment(s).

Embodiments of the present disclosure are to provide an air conditioner and control method thereof for sterilizing an indoor unit fan.

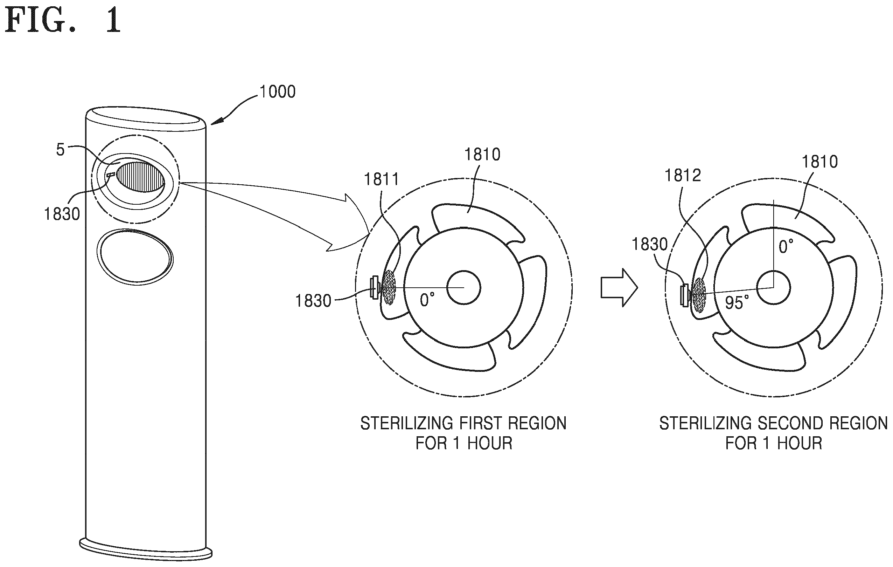

FIG. 1 illustrates a method, performed by an air conditioner, of sterilizing an indoor unit fan, according to an embodiment of the present disclosure.

Referring to FIG. 1, the air conditioner 1000 may sterilize a stopped fan after finishing a cooling operation. The air conditioner 1000 may sterilize a partial region of the stopped fan and then store location information of the sterilized region. In addition, the air conditioner 1000 may temporarily rotate the fan to sterilize another region of the fan. When the temporarily rotated fan stops, the air conditioner 1000 may determine whether a region being irradiated with ultraviolet (UV) light is a sterilized region. When the region being irradiated with UV light is an non-sterilized region, the air conditioner 1000 may sterilize the region being irradiated with UV light, and when the region being irradiated with UV light is a sterilized region, the air conditioner 1000 may temporarily rotate the fan again to sterilize another region of the fan.

For example, as illustrated in FIG. 1, the air conditioner 1000 may not apply a driving current to a fan motor (not shown) based on a user input for ending the cooling operation. Even when the driving current is not applied to the fan motor (not shown), the fan 1810 may rotate and then gradually stop due to rotational inertia.

In addition, the air conditioner 1000 may sterilize a partial region 1811 of the stopped fan 1810. A UV light output module 1830 may be provided in a space 5 in which the fan 1810 within the air conditioner 1000 is housed, and may sterilize the partial region 1811 of the stopped fan 1810 by irradiating UV light onto the partial region 1811 of the fan 1810 for at least a reference time (e.g., for at least 1 hour).

Furthermore, the air conditioner 1000 may rotate the fan 1810 to sterilize another region of the fan 1810 after sterilizing the partial region 1811 of the fan 1810. The air conditioner 1000 may apply a driving voltage to the fan motor for a driving time (e.g., 1 second) for changing a region to be sterilized (also hereinafter, a sterilization region), based on a revolutions per minute (RPM) (e.g., 50 rpm) for changing the sterilization region, and then may not apply a driving voltage thereto. According to an embodiment, the RPM for changing the sterilization region may be lower than a lowest RPM for the cooling operation.

After the fan 1810 stops rotating, the air conditioner 1000 may determine whether a region 1812 being irradiated with UV light is a region that has already been sterilized. When the region being irradiated with UV light is a region that has already been sterilized, the air conditioner 1000 may rotate the fan 1810 again to sterilize another region of the fan 1810. According to an embodiment, the air conditioner 1000 may more easily change a sterilization region by driving the fan motor at a different RPM than a previous RPM. Furthermore, when the region 1812 being irradiated with UV light is a region that is different from the partial region 1811 that has previously been sterilized, the air conditioner 1000 may sterilize the region 1812 being irradiated with UV light by irradiating the region 1812 with UV light for the reference time or more.

The air conditioner 1000 may store location information of the region 1812 that is irradiated with UV light after sterilization. Thereafter, the air conditioner 1000 may determine, based on the stored location information, whether a partial region of the fan 1810 is a sterilized region.

According to an embodiment, the air conditioner 1000 may include an illuminance sensor (not shown), together with the UV light output module 1830, in the space 5 in which the fan 1810 is provided. In addition, the air conditioner 1000 may receive, via the illuminance sensor (not shown), light reflected from the fan 1810), and determine an intensity of the received reflected light. The air conditioner 1000 may store an intensity of reflected light from a region that is irradiated with UV light as location information of the region irradiated with UV light.

Furthermore, according to an embodiment, the air conditioner 1000 may store a sensor value of Hall sensors in the fan motor as location information of a region being irradiated with UV light.

According to an embodiment, the air conditioner 1000 may sterilize the fan 1810 by driving the UV light output module 1830 when the fan 1810 stops based on a user input for stopping a cooling operation.

According to an embodiment, in response to receiving a user input for starting a cooling operation, the air conditioner 1000 may stop driving the UV light output module 1830 and terminate sterilization of the fan 1810.

According to an embodiment, the air conditioner 1000 may start sterilizing the fan 1810 by driving the UV light output module 1830 based on a user input for starting the sterilization of the fan 1810. According to an embodiment, the air conditioner 1000 may terminate the sterilization of the fan 1810 by not driving the UV light output module 1830 based on a user input for terminating the sterilization of the fan 1810.

The air conditioner 1000 may remove bacteria attached to the fan 1810 by irradiating the fan 1810 with UV light. However, because only a portion that is directly irradiated is sterilized due to the properties of UV light having directionality, in order to sterilize the entire fan 1810, the entire fan 1810 has to be irradiated with UV light, or the fan 1810 needs to be continuously rotated.

Furthermore, even when UV light is irradiated onto the fan 1810, the bacteria are not immediately removed, and the UV light needs to be irradiated for a sufficient period of time to remove most of the bacteria. For example, when UV light is continuously irradiated onto the rotating fan 1810 during a cooling operation, 99.99% of the bacteria may be removed only when UV irradiation time is at least 4 hours. Also, for example, when UV light is irradiated onto the stopped fan 1810, a UV irradiation time of 1 hour or more needs to elapse before most of the bacteria in the irradiated region can be removed.

Thus, in order to irradiate the entire fan 1810 with UV light for a sufficient period of time, the number of UV light sources may be increased or the size of the light sources may be increased, but this may significantly increase manufacturing costs. Furthermore, when a cooling operation time is short even though UV light is continuously irradiated onto the rotating fan 1810 during the cooling operation, the sterilization may be terminated without the bacteria being removed, which allows the bacteria to proliferate again. Furthermore, when the fan 1810 continues to rotate after the air conditioner 1000 stops operating, an airflow may be generated, and even a weak airflow may cause discomfort to a user who wants to stop cooling.

The air conditioner 1000 may sterilize the entire fan 1810 without causing discomfort to the user by changing a sterilization region via temporary rotation of the fan 1810 after the end of the cooling operation. In addition, even when the cooling operation time is not sufficient to sterilize most of the bacteria, most of the bacteria may be sterilized after the end of the cooling operation.

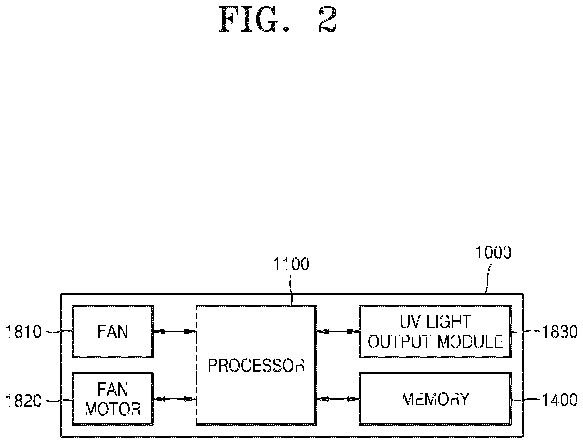

FIG. 2 is a block diagram of an air conditioner according to an embodiment of the present disclosure.

Referring to FIG. 2, an air conditioner 1000 may include a processor 1100, a fan 1810, a fan motor 1820, a UV light output module 1830, and a memory 1400.

The processor 1100 may generally control all operations of the air conditioner 1000. The processor 1100 may execute programs stored in the memory 1400 to control the fan 1810, the fan motor 1820, and the UV light output module 1830.

The air conditioner 1000 may include a device that forms an air flow by using the fan 1810. For example, the air conditioner 1000 may rotate the fan 1810 to draw in outside air. Also, for example, the air conditioner 1000 may rotate the fan 1810 to discharge cooled air to the outside. For example, the air conditioner 1000 may include, but is not limited to, an air handler, an air purifier, a ventilator, an electric fan, a ventilation system, and a heat exchanger.

The memory 1400 stores various pieces of information, data, instructions, programs, etc. necessary for the operations of the air conditioner 1000. The memory 1400 may include at least one of volatile memory or non-volatile memory, or a combination thereof.

The fan 1810 may be a device that forms a flow of air while rotating about a rotation axis. The fan 1810 may include axial fans, such as a propeller fan, and centrifugal fans, such as a turbo fan, a plate fan, and a Sirocco fan. The air conditioner 1000 may include a single fan 1810, or a plurality of fans 1810 of various types. Blades of the fan 1810 may respectively have reflective tapes with different reflectivities attached thereto.

The fan motor 1820 may be connected to the rotation axis of the fan 1810 to rotate the fan 1810. The fan motor 1820 may include, but is not limited to, a brushless direct current (BLDC) motor, a DC motor, or an alternating current (AC) motor. The fan motor 1820 may include at least one Hall sensor (not shown).

When the air conditioner 1000 starts to operate, the processor 1100 may apply a driving voltage to the fan motor 1820. When the driving voltage is applied to the fan motor 1820, the fan motor 1820 is able to rotate.

When the fan motor 1820 rotates, the fan 1810 connected to the fan motor 1820 may rotate accordingly, and while the fan 1810 rotates, outside air may be drawn into the air conditioner 1000, or cooled air inside the air conditioner 1000 may be discharged to the outside.

The UV light output module 1830 may include a UV light-emitting diode (LED) that emits UV light. The UV LED may include, for example, a UV-A LED and a UV-C LED. The UV light output module 1830 may be located on a side of the fan 1810, but is not limited thereto. Additionally, one UV light output module 1830 may be provided for one fan 1810, and a plurality of UV light output modules 1830 may be provided for one fan 1810.

The processor 1100 may execute one or more instructions stored in the memory 1400 to control the UV light output module 1830 to irradiate UV light onto the fan 1810.

Furthermore, when the fan 1810 stops rotating, the processor 1100 may determine whether a partial region of the fan 1810 irradiated with UV light is a region sterilized by the UV light output module 1830.

In addition, when determining that the partial region is a sterilized region, the processor 1100 may sterilize another region of the fan 1810 by driving the fan motor 1820 to rotate the fan 1810.

In addition, when determining that the partial region is a non-sterilized region, the processor 1100 may sterilize the partial region by irradiating the region with UV light for a predetermined sterilization time.

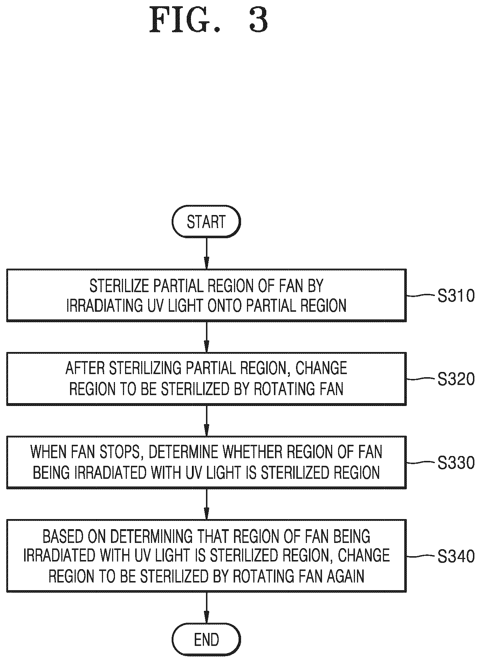

FIG. 3 is a flowchart of a method, performed by an air conditioner, of sterilizing an indoor unit fan, according to an embodiment of the present disclosure.

In operation S310, the air conditioner 1000 may sterilize a partial region of the fan 1810 by irradiating UV light onto the partial region.

When a cooling operation of the air conditioner 1000 is finished and the fan 1810 stops rotating, the air conditioner 1000 may sterilize a partial region of the fan 1810 by irradiating UV light onto the partial region for a predetermined sterilization time.

The air conditioner 1000 may control the UV light output module 1830 to irradiate UV light onto the fan 1810. UV light emitted by the UV light output module 1830 may be irradiated onto a partial region of the fan 1810. For example, when the UV light output module 1830 is provided on a side of the fan 1810, UV light emitted by the UV light output module 1830 may be irradiated onto an angular range of the fan 1810.

After irradiating the UV light for the predetermined sterilization time, the air conditioner 1000 may store a location of the sterilized partial region as location information of the sterilized region.

For example, the air conditioner 1000 may store an intensity of light reflected by a reflective material attached to the fan 1810 as location information of the sterilized region.

In addition, for example, the air conditioner 1000 may store a last sensor value of the Hall sensor before the fan 1810 stops as location information of the sterilized region.

In operation S320, after sterilizing the partial region, the air conditioner 1000 may change a region to be sterilized by rotating the fan.

After storing the location of the sterilized partial region as the location information of the sterilized region, the air conditioner 1000 may temporarily rotate the fan 1810 in order to change a sterilization region. For example, the air conditioner 1000 may drive the fan motor for a driving time for changing a sterilization region, based on an RPM for changing the sterilization region. The air conditioner 1000 may drive the fan motor by applying a driving voltage corresponding to the RPM for changing the sterilization region to the fan motor for the driving time for changing the sterilization region. Furthermore, the air conditioner 1000 may drive the fan motor by applying a driving current corresponding to the RPM for changing the sterilization region to the fan motor for the driving time for changing the sterilization region.

In operation S330, when the fan 1810 stops, the air conditioner 1000 may determine whether a region of the fan 1810 being irradiated with UV light is a sterilized region.

After the driving time for changing the sterilization region has lapsed, according to a driving voltage not being applied to the fan motor 1820, a central axis of the fan motor 1820 may gradually stop due to rotational inertia.

When the fan 1810 stops, the air conditioner 1000 may determine whether a partial region of the fan 1810 being irradiated with UV light is a sterilized region.

For example, the air conditioner 1000 may receive, via the illuminance sensor, light reflected by a reflective material attached to the fan 1810, and, based on a difference between an intensity of the received reflected light and the intensity of reflected light stored as the location information of the sterilized region, determine whether a partial region of the fan 1810 being irradiated with UV light is a region sterilized by the UV light output module 1830. The blades of the fan 1810 may have reflective materials with different reflectivities from each other attached thereto.

In addition, for example, the air conditioner 1000 may determine whether a partial region of the fan 1810 being irradiated with UV light is a region sterilized by the UV light output module 1830 by comparing a last sensor value of the Hall sensor before the fan 1810 stops with the sensor value stored as the location information of the sterilized region.

In addition, for example, the air conditioner 1000 may determine whether a partial region of the fan 1810 being irradiated with UV light is a region sterilized by the UV light output module 1830, based on a sensor value of the Hall sensor as well as an intensity of reflected light detected via the illuminance sensor.

In operation S340, based on determining that the region of the fan being irradiated with the UV light is a sterilized region, the air conditioner 1000 may change a region to be sterilized by rotating the fan 1810.

Based on determining that the partial region is a sterilized region, the air conditioner 1000 may change a region to be sterilized by driving the fan motor 1820 to rotate the fan 1810. In this case, the air conditioner 1000 may drive the fan motor 1820 based on an RPM lower than a lowest RPM for a cooling operation. In addition, the air conditioner 1000 may drive the fan motor 1820 based on an RPM that is different from the previous RPMs each time it drives the fan motor 1820 to change the sterilization region.

Based on determining that the region of the fan 1810 being irradiated with the UV light is a sterilized region, the air conditioner 1000 may change a region to be sterilized by driving the fan motor 1820 to rotate the fan 1810 again, and based on determining that the region of the fan 1810 being irradiated with UV light is a non-sterilized region, sterilize the region of the fan 1810 being irradiated with the UV light by irradiating UV light onto the region of the fan 1810 for a predetermined sterilization time.

According to an embodiment, the air conditioner 1000 may start sterilizing the fan 1810 when the fan 1810 stops based on a user input for stopping the cooling operation. Furthermore, in an embodiment, the air conditioner 1000 may terminate the sterilization of the fan 1810 based on a user input for starting the cooling operation of the air conditioner 1000.

According to an embodiment, the air conditioner 1000 may start sterilizing the fan 1810 based on a user input for starting the sterilization of the fan 1810. In addition, according to an embodiment, the air conditioner 1000 may terminate sterilization of the fan 1810 based on a user input for terminating the sterilization of the fan 1810.

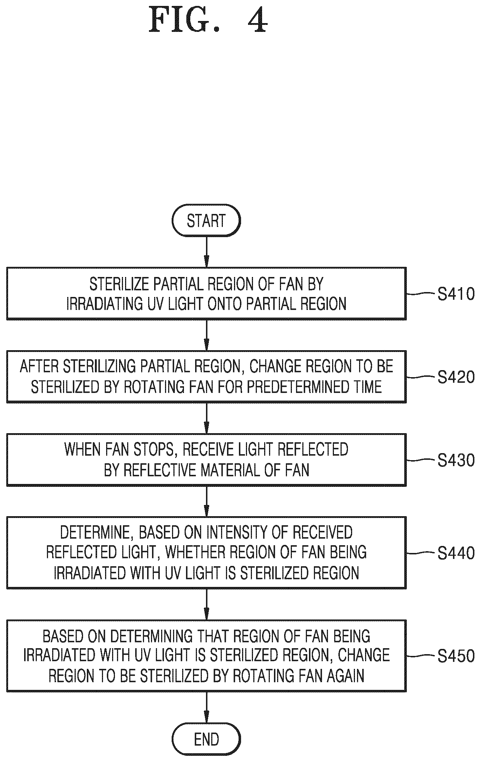

FIG. 4 is a flowchart of a method, performed by an air conditioner, of determining a location of a partial region of a fan being irradiated with UV light based on an intensity of the light reflected by the fan, according to an embodiment of the present disclosure.

In operation S410, the air conditioner 1000 may sterilize a partial region of the fan 1810 by irradiating UV light onto the partial region.

After irradiating the UV light for the predetermined sterilization time, the air conditioner 1000 may store a location of the sterilized partial region as location information of the sterilized region.

For example, the air conditioner 1000 may store an intensity of light reflected by a reflective material attached to the fan 1810 as location information of the sterilized region.

Furthermore, for example, the air conditioner 1000 may identify a sterilized blade or angular range and store the identified blade or angular range as location information of the sterilized region. The air conditioner 1000 may identify a blade being irradiated with UV light, based on an intensity of reflected light received from the fan 1810 via the illuminance sensor. In addition, the air conditioner 1000 may determine an angular range of the fan 1810 on which UV light is being irradiated, based on an intensity of reflected light received from the fan 1810 via the illuminance sensor.

In operation S420, after sterilizing the partial region of the fan 1810, the air conditioner 1000 may change a region to be sterilized by rotating the fan 1810 for a predetermined time. Operation S420 may be described with reference to operation S320 of FIG. 3.

In operation S430, when the fan 1810 stops, the air conditioner 1000 may receive light reflected by a reflective material of the fan 1810.

A reflective tape or reflective film with a different reflectivity (albedo, expressed as a percentage (%)) may be attached to each of the blades of the fan 1810. Furthermore, each of the blades of the fan 1810 of the air conditioner 1000 may be coated with a reflective dye having a different reflectivity.

Furthermore, reflective dyes may be applied to the fan 1810 so that the intensity of reflected UV light being irradiated onto the fan 1810 increases as an angle increases with a vertex at a center of the fan 1810.

In addition, a reflector having a different reflectivity may be attached to each of the blades of the fan 1810. In addition, the fan 1810 may include different reflective structures on each blade thereof.

According to the UV light irradiated from a UV light output module 1830 being reflected by the reflective material of the fan 1810, the air conditioner 1000 may receive the reflected light. In this case, an intensity of reflected light may vary depending on a rotation angle of the fan 1810.

In operation S440, the air conditioner 1000 may determine, based on an intensity of the received reflected light, whether a region of the fan 1810 being irradiated with UV light is a sterilized region.

According to an embodiment, when the fan 1810 stops again after rotating the fan 1810 to change a region to be sterilized, the air conditioner 1000 may identify again a blade or angular range where the UV light is being irradiated. In addition, the air conditioner 1000 may determine whether the region being irradiated with UV light is a sterilized region based on whether the identified blade is the same as identification information of the blade stored as the location information of the sterilized region, or whether a difference between the identified angular range and the angular range stored as the location information of the sterilized region is within a reference angular range.

According to an embodiment, the air conditioner 1000 may determine that the region being irradiated with UV light is a sterilized region when a difference between the intensity of reflected light received from the fan 1810 and the intensity of reflected light stored as the location information of the sterilized region is less than a reference light intensity.

In operation S450, based on determining that the region of the fan 1810 being irradiated with the UV light is a sterilized region, the air conditioner 1000 may change the region to be sterilized by rotating the fan 1810 again. Operation S450 may be described with reference to operation 340 of FIG. 3.

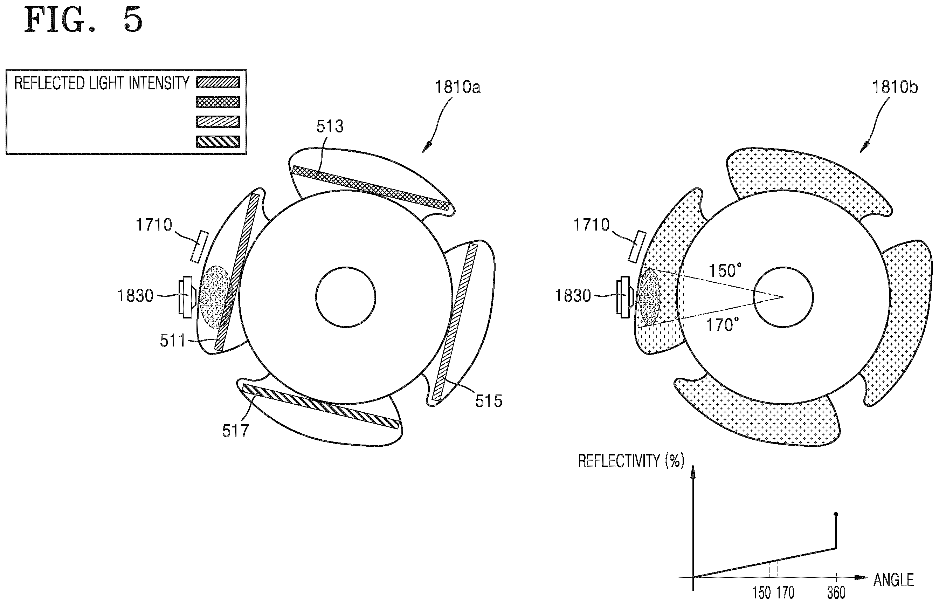

FIG. 5 illustrates a method, performed by an air conditioner, of determining a location of a partial region of a fan being irradiated with UV light based on intensity of light reflected by the fan, according to an embodiment of the present disclosure.

Referring to FIG. 5, a reflective tape or reflective film having a different reflectivity may be attached to each of the blades of the air conditioner 1000. Furthermore, according to an embodiment, a reflective dye having a different reflectivity may be applied to each of the blades of the fan 1810 of the air conditioner 1000.

According to an embodiment, as shown in a diagram on the left of FIG. 5, blades of a fan 1810a may respectively have reflective tapes 511, 513, 515, and 517 with different reflectivities attached thereto.

Furthermore, according to an embodiment, a reflective dye having a different reflectivity may be applied to each of the blades of the fan 1810a. For example, a reflective dye having a first reflectivity may be applied to an entire first blade, and a reflective dye having a second reflectivity may be applied to an entire second blade. Furthermore, according to an embodiment, a reflector having a different reflectivity may be attached to each of the blades of the fan 1810a. In addition, according to an embodiment, the fan 1810a may include different reflective structures on each blade thereof. For example, each blade may be provided with a reflective protrusion having a different shape.

In addition, the air conditioner 1000 may store an intensity of reflected light corresponding to identification information of a blade. Accordingly, the air conditioner 1000 may identify a blade that is being irradiated with UV light, based on an intensity of reflected light received from the fan 1810a via an illuminance sensor 1710.

As shown in a diagram on the right of FIG. 5, the right fan 1810b of FIG. 5, a reflective dye may be applied to an entire fan 1810b so that the reflectivity increases as an angle increases with a center of the fan 1810b as a center point. In addition, the air conditioner 1000 may store an intensity of reflected light corresponding to an angle of a region being irradiated with UV light. Accordingly, the air conditioner 1000 may control the UV light output module 1830 to irradiate UV light onto the fan 1810b, and determine an angular range onto which the UV light is being irradiated, based on an intensity of reflected light received from the fan 1810b via the illuminance sensor 1710. For example, the air conditioner 1000 may determine, based on the intensity of reflected light received from the fan 1810b, the angular range of the fan 1810b onto which UV light is being irradiated as being 150 degrees to 170 degrees.

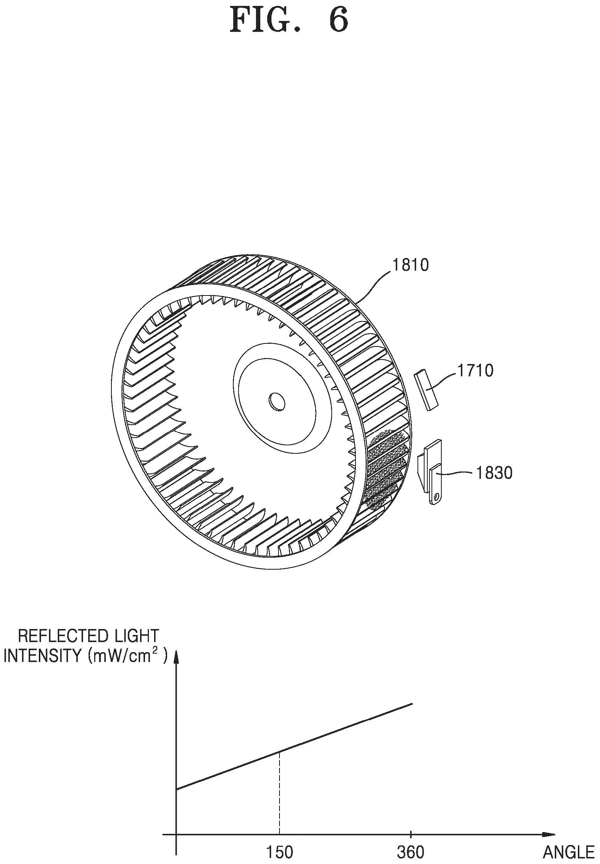

FIG. 6 illustrates a method, performed by an air conditioner, of determining whether a partial region of a fan being irradiated with UV light is a sterilized region based on an intensity of reflected light, according to an embodiment of the present disclosure.

Referring to FIG. 6, a reflective dye may be applied to the fan 1810 such that the intensity of reflected UV light being irradiated onto the fan 1810 increases as an angle increases with a center point at a center of the fan 1810. When the fan 1810 stops due to the end of the cooling operation, the air conditioner 1000 may irradiate UV light onto an angular range of the fan 1810 for a predetermined sterilization time, and store an intensity of reflected light received from the angular range as a first light intensity. In addition, when the fan 1810 stops after rotating the fan the air conditioner 1000 may receive reflected light from the fan 1810 and detect an intensity of the reflected light. Based on determining that a difference between the detected light intensity and the first light quantity is greater than or equal to a reference light intensity, the air conditioner 1000 may determine that an angular range of the fan 1810 being irradiated with UV light is a non-sterilized region.

Accordingly, even when the air conditioner 1000 does not determine an angle of the region being irradiated with UV light, the air conditioner 1000 may determine whether the region being irradiated with the UV light is a sterilized region based on light intensities stored in correspondence to the previously sterilized partial regions of the fan 1810.

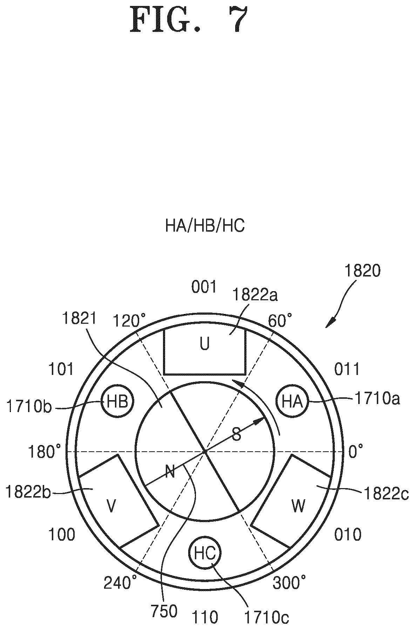

FIG. 7 illustrates a method, performed by an air conditioner, of determining whether a partial region of a fan being irradiated with UV light is a sterilized region based on a sensor value of a Hall sensor, according to an embodiment of the present disclosure.

Referring to FIG. 7, the fan motor 1820 of the air conditioner 1000 may be a BLDC motor.

The BLDC motor 1820 may include a rotor 1821, composed of a permanent magnet, and stators, 1822a, 1822b, and 1822c, having magnet wires wound around them. The rotor 1821 may be composed of a dipole magnet including an north (N) pole and a south (S) pole, as shown in FIG. 7, or may be composed of a four-pole, six-pole, eight-pole, or ten-pole magnet including a plurality of pairs of N- and S-poles. The air conditioner 1000 may rotate the rotor 1821 in one direction by periodically changing voltage values of the stators 1822a, 1822b, and 1822c according to an angle and a polarity of the rotor 1821.

The BLDC motor 1820 may include three Hall sensors 1710a, 1710b, and 1710c, and the three Hall sensors may be positioned on the fan motor 1820 at intervals of 120 degrees. Each of the Hall sensors 1710a, 1710b, and 1710c may be a sensor that outputs a value of 0 when the S pole of the magnet approaches it and a value of 1 when the N pole of the magnet approaches it.

Furthermore, the angle and polarity of rotor 1821 may be matched to an angle and a polarity of an arrow 750 which passes through a center of the rotor 1821 and indicates a direction from the N pole to the S pole. The air conditioner 1000 may detect the angle and polarity of the rotor 1821 by using the three Hall sensors 1710a, 1710b, and 1710c.

For example, when the arrow 750 rotates counterclockwise from 0 degrees to 60 degrees, the first Hall sensor 1710a may output the value of 0 because the S pole approaches the first Hall sensor 1710a, the second Hall sensor 1710b may output the value of 1 because the N pole approaches the second Hall sensor 1710b, and the third Hall sensor 1710c may output the value of 1 because the N pole approaches the third Hall sensor 1710c. Therefore, while the arrow 750 rotates counterclockwise from 0 degrees to 60 degrees, the first Hall sensor 1710a, the second Hall sensor 17210b, and the third Hall sensor 1710c may output a value of 011.

Furthermore, for example, when the arrow 750 rotates counterclockwise from 60 degrees to 120 degrees, the first Hall sensor 1710a may output the value of 0 because the S pole approaches the first Hall sensor 1710a, the second Hall sensor 1710b may output the value of 0 because the S pole approaches the second Hall sensor 1710b, and the third Hall sensor 1710c may output the value of 1 because the N pole approaches the third Hall sensor 1710c. Therefore, while the arrow 750 rotates counterclockwise from 60 degrees to 120 degrees, the first Hall sensor 1710a, the second Hall sensor 17210b, and the third Hall sensor 1710c may output a value of 001.

When the rotor 1821 stops after rotating, the air conditioner 1000 may determine an angle of the rotor 1821 after it stops, based on a sensor value of the three Hall sensors 1710a, 1710b, and 1710c. For example, when a last value of the three Hall sensors 1710a, 1710b, and 1710c is a value of "011", the air conditioner 1000 may identify that the rotor 1821 has stopped between 0 degrees and 60 degrees.

In addition, FIG. 7 illustrates that when the rotor 1821 has 2 poles, an identifiable angular range of the rotor is 60 degrees. The rotor 1821 may have various poles, such as 4 poles, 6 poles, 8 poles, 10 poles, etc., and as the number of poles of the rotor 1821 increase, an identifiable angular range of the rotor 1821 may become more precise. For example, an identifiable angular range of the rotor may be 45 degrees when the rotor 1821 has 4 poles, 30 degrees when the rotor 1821 has 6 poles, and 15 degrees when the rotor 1821 has 8 poles.

When the fan motor 1820 stops, the air conditioner 1000 may sterilize a partial region of the fan 1810, and store a last sensor value of the Hall sensors as location information of the sterilized region. In addition, the air conditioner 1000 may temporarily rotate the fan 1810 again to change a sterilization region, and when the fan 1810 stops again, the air conditioner 1000 may determine whether a region being irradiated with UV light is a sterilized region by comparing a last sensor value of the Hall sensors with the location information of the sterilized region.

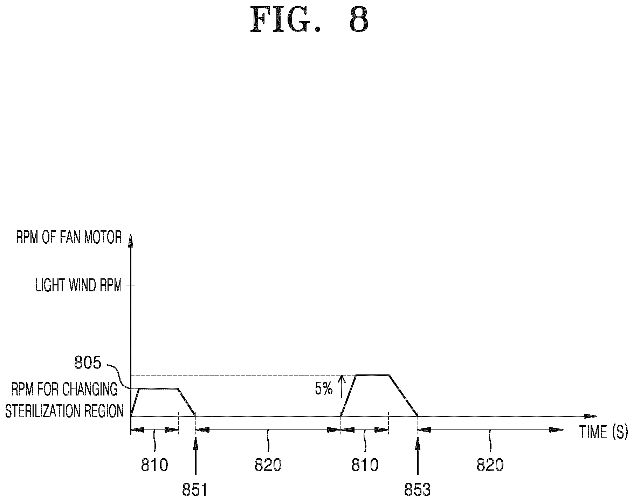

FIG. 8 illustrates a method, performed by an air conditioner, of changing a sterilization region of a fan, according to an embodiment of the present disclosure.

Referring to FIG. 8, when driving the fan motor 1820 to change a sterilization region, the air conditioner 1000 may drive the fan motor 1820 at a different RPM than a previous RPM.

Referring to a graph of FIG. 8, the air conditioner 1000 may drive the fan motor 1820 for a driving time 810 for changing a sterilization region, based on an RPM 805 for changing the sterilization region after sterilizing a partial region of the fan 1810. The RPM 805 for changing the sterilization region may be lower than a minimum RPM (e.g., a light wind RPM) used in a cooling operation of the air conditioner 1000.

The driving of the fan motor 1820 for changing the sterilization region is not for generating wind, but for changing an angle at which the fan 1810 stops, and because the driving is performed while the air conditioner 1000 has stopped operation thereof, the RPM 805 for changing the sterilization region may be determined to be an RPM (e.g., 50 RPM) that is too low for the user to recognize the driving of the fan motor 1820.

The driving time 810 for changing the sterilization region may also be determined to be a time period (e.g.. 1 second) that is too short for the user to recognize the driving of the fan motor 1820.

At a time point 851 when the fan 1810 stops after the fan motor 1820 is driven for the driving time 810 for changing the sterilization region, the air conditioner 1000 may determine whether a region of the fan 1810 being irradiated with UV light is a sterilized region. When the partial region of the fan 1810 being irradiated with the UV light is a non-sterilized region, the air conditioner 1000 may sterilize the region of the fan 1810 by controlling the UV light output module 1830 to irradiate UV light onto the fan 1810 for a predetermined sterilization time 820.

When the sterilization of the region of the fan 1810 is completed, the air conditioner 1000 may drive the fan motor 1820 again for the driving time 810 for changing the sterilization region. In this case, the air conditioner 1000 may drive the fan motor 1820 based on a different RPM than the previous RPMs 805 for changing the sterilization region. For example, the air conditioner 1000 may drive the fan motor 1820 based on an RPM that is a predetermined percentage (e.g., 5 %) higher than the previous RPM each time the fan motor 1820 is driven for changing the sterilization region. Accordingly, the air conditioner 1000 may induce sterilization of a different region by UV light each time it drives the fan motor 1820.

At a time point 853 when the fan 1810 stops again after the fan motor 1820 is driven for the driving time 810 for changing the sterilization region, the air conditioner 1000 may determine whether another partial region of the fan 1810 being irradiated with UV light is a sterilized region. When the other partial region of the fan 1810 being irradiated with the UV light is a non-sterilized region, the air conditioner 1000 may sterilize the other partial region of the fan 1810 by controlling the UV light output module 1830 to irradiate UV light onto the fan 1810 for the predetermined sterilization time 820.

The air conditioner 1000 may store information indicating locations of the sterilized partial regions, and may determine whether all regions of the fan 1810 have been sterilized based on the information indicating the locations of the sterilized partial regions. According to determining that all of the regions of the fan 1810 have been sterilized, the air conditioner 1000 may terminate a sterilization mode without further driving the fan motor 1820.

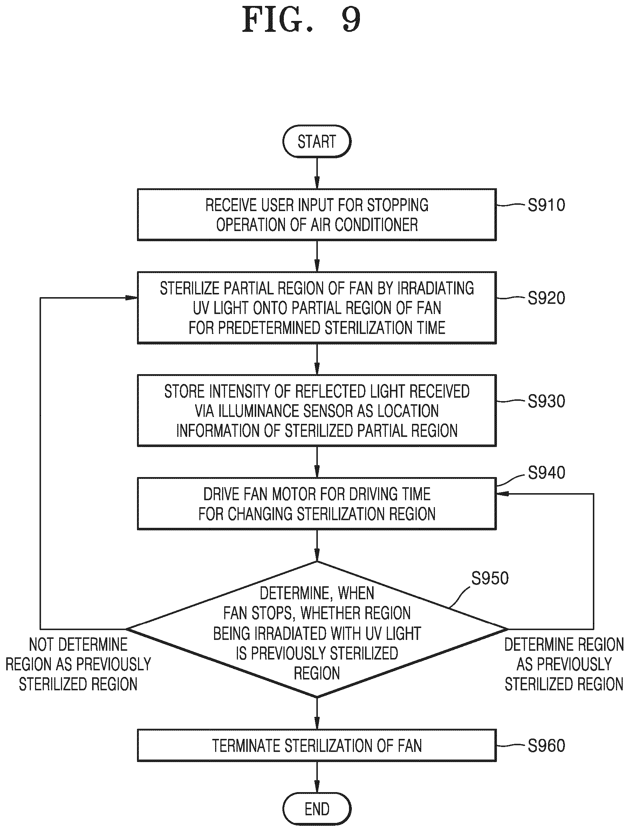

FIG. 9 is a flowchart of a method, performed by an air conditioner, of sterilizing a fan, according to an embodiment of the present disclosure.

In operation S910, the air conditioner 1000 may receive a user input for stopping operation of the air conditioner 1000.

The air conditioner 1000 may receive a user input for stopping operation of the air conditioner 1000 during the operation.

In operation S920, the air conditioner 1000 may sterilize a partial region of the fan 1810 by irradiating UV light onto the partial region for a predetermined sterilization time.

Based on the user input for stopping the operation, the fan 1810 may gradually stop according to a driving voltage not being applied to the fan motor 1820. When the fan 1810 stops, the air conditioner 1000 may sterilize a partial region of the fan 1810 by irradiating UV light for the predetermined sterilization time.

In operation S930, the air conditioner 1000 may store an intensity of reflected light received via the illuminance sensor as location information of the sterilized partial region.

After sterilizing the partial region of the fan 1810 by irradiating the UV light for the predetermined sterilization time, the air conditioner 1000 may store an intensity of reflected light received via the illuminance sensor as location information of the sterilized region.

In operation S940, the air conditioner 1000 may drive the fan motor 1820 for a driving time for changing a sterilization region.

In order to change the sterilization region, the air conditioner 1000 may drive the fan motor 1820 for the driving time for changing the sterilization region.

In operation S950, when the fan 1810 stops, the air conditioner 1000 may determine whether a region being irradiated with UV light is a previously sterilized region.

When the fan 1810 stops after the fan motor 1820 is driven for the driving time for changing the sterilization region, the air conditioner 1000 may determine whether a region being irradiated with UV light is a previously sterilized region based on a difference between an intensity of reflected light received via the illuminance sensor and the intensity of reflected light stored as the location information of the sterilized partial region.

For example, when the difference between the intensity of reflected light being received and the intensity of reflected light stored as the location information is greater than or equal to a reference light intensity difference, the air conditioner 1000 may determine that the region being irradiated with the UV light is not a previously sterilized region, and when the difference is less than the reference light intensity difference, determine that the region being irradiated with the UV light is a previously sterilized region.

Based on determining in operation S950 that the region being irradiated with the UV light is not a previously sterilized region, the air conditioner 1000 may return to operation S920 and sterilize the region being irradiated with the UV light by irradiating the region with UV light for the predetermined sterilization time. In addition, based on determining in operation S950 that the region being irradiated with the UV light is a previously sterilized region, the air conditioner 1000 may return to operation S940 and drive the fan motor 1820 again for the driving time for changing the sterilization region.

When the region being irradiated with the UV light is a previously sterilized region in operation S950, and a predetermined total sterilization time (e.g., 7 hours) has elapsed, the air conditioner 1000 may terminate the sterilization of the fan 1810 in operation S960.

Furthermore, according to determining that the region being irradiated with the UV light is a previously sterilized region in operation S950 and that all regions of the fan 1810 are determined to have been sterilized based on location information of the previously sterilized regions, the air conditioner 1000 may terminate the sterilization of the fan 1810 in operation S960.

In addition, according to receiving a user input for starting the operation of the air conditioner 1000, in operation S960, the air conditioner 1000 may terminate the sterilization of the fan 1810 and start the operation thereof. For example, the air conditioner 1000 may start cooling and heating operations without further applying a driving voltage to the UV light output module 1830.

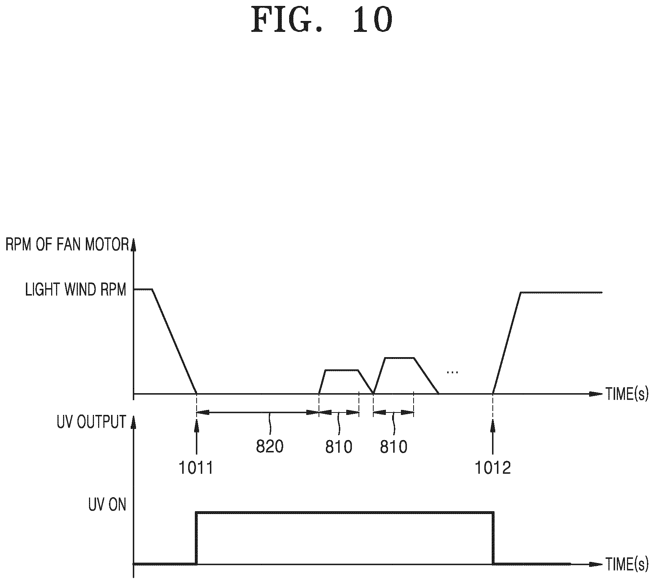

FIG. 10 illustrates a method, performed by an air conditioner, of starting sterilization of a fan, according to an embodiment of the present disclosure.

According to an embodiment, as illustrated in FIG. 10, the air conditioner 1000 may start a sterilization mode when a cooling operation is terminated. In addition, the sterilization mode may be terminated when the cooling operation starts during the sterilization mode.

The air conditioner 1000 may receive a user input for terminating the cooling operation during the cooling operation. Based on the user input for terminating the cooling operation, the air conditioner 1000 may not apply a driving voltage to the fan motor 1820.

According to detection that rotation of the fan 1810 has stopped, the air conditioner 1000 may start a sterilization mode sterilizing the fan 1810. For example, at a time point 1011 when the sterilization mode is started, the air conditioner 1000 may apply a voltage to the UV light output module 1830 to output UV light, and sterilize a partial region of the fan 1810 being irradiated with UV light by irradiating the partial region with the UV light for a sterilization time 820. After sterilizing the partial region, the air conditioner 1000 may drive the fan motor 1820 for a driving time 810 for changing a sterilization region. When the fan 1810 stops after the fan motor 1820 is driven, the air conditioner 1000 may determine whether a partial region being irradiated with UV light is a sterilized region. When the partial region being irradiated with the UV light is a sterilized region, the air conditioner 1000 may drive the fan motor 1820 again for the driving time 810 for changing the sterilization region based on a different RPM than a previous RPM.

Based on a user input for starting a cooling operation while sterilizing the fan 1810 in the sterilization mode, the air conditioner 1000 may terminate the sterilization mode. From a time point 1012 when the sterilization mode is terminated, the air conditioner 1000 may drive the fan motor 1820 based on an RPM for the cooling operation without applying a voltage to the UV light output module 1830.

According to an embodiment, the air conditioner 1000 may also sterilize the fan 1810 continuously even during the cooling operation. When receiving a user input for terminating the cooling operation while continuously sterilizing the fan 1810 during the cooling operation, the air conditioner 1000 may continuously apply a driving voltage to the UV light output module 1830 without stopping the UV output. After terminating the cooling operation, the air conditioner 1000 may sterilize partial regions of the fan 1810 by temporarily rotating the fan 1810, and when all regions of the fan 1810 are sterilized, the air conditioner 1000 may terminate the sterilization mode.

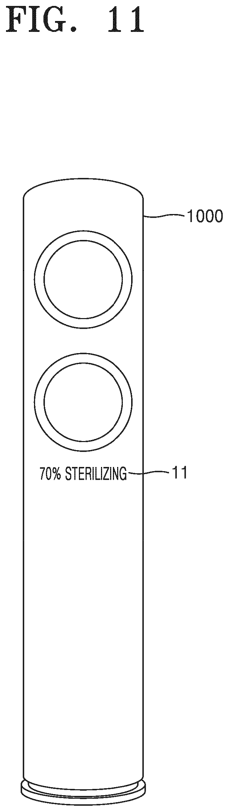

FIG. 11 illustrates a method, performed by an air conditioner, of indicating that sterilization is being performed, according to an embodiment of the present disclosure.

Referring to FIG. 11, when irradiating the UV light by applying a driving voltage to the UV light output module 1830, the air conditioner 1000 may display a phrase or image indicating that sterilization is being performed.

In addition, after the operation of the air conditioner 1000 is ended, according to sterilizing partial regions of the fan 1810 by temporarily rotating the fan 1810, the air conditioner 1000 may display a phrase or image indicating a degree of progress of the sterilization. For example, the air conditioner 1000 may calculate a degree of progress of sterilization based on a ratio of sterilized partial regions to the entire region of the fan 1810, and display the calculated degree of progress of sterilization.

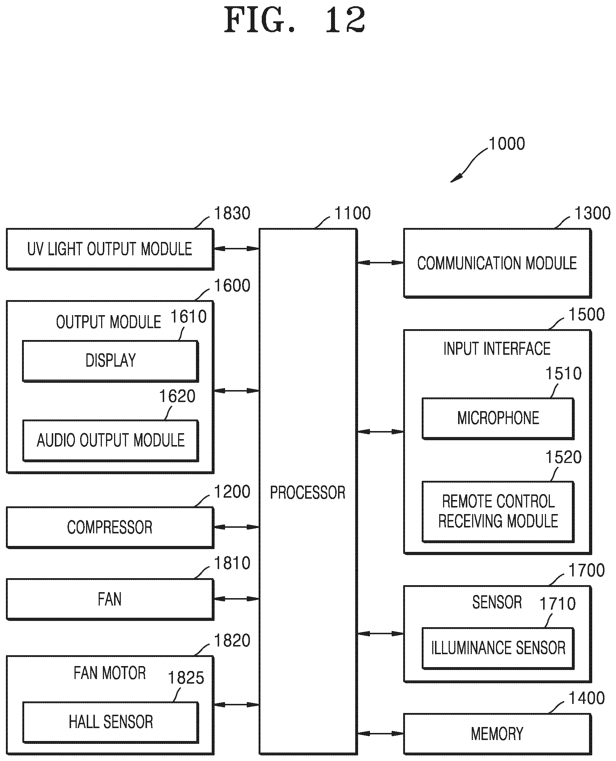

FIG. 12 is a block diagram of an air conditioner according to an embodiment of the present disclosure.

Referring to FIG. 12, the air conditioner 1000 may include the processor 1100, the UV light output module 1830, an output module 1600, a compressor 1200, the fan 1810, the fan motor 1820, a communication module 1300, an input interface 1500, a sensor 1700, and the memory 1400.

However, all of the components shown in FIG. 12 are not essential components of the air conditioner 1000. The air conditioner 1000 may be implemented by more components than those shown in FIG. 12, or by fewer components than those shown in FIG. 12. For example, the air conditioner 1000 may be implemented with only the processor 1100, the UV light output module 1830, the compressor 1200, the fan 1810, the fan motor 1820, the input interface 1500, and the memory 1400.

The fan 1810 may form an airflow to draw outside air into the air conditioner 1000. The fan 1810 may also discharge air cooled by the heat exchanger to the outside of the air conditioner 1000. The fan 1810 may be rotated by the fan motor 1820 to create an airflow. A rotation speed (i.e., an RPM) of the fan motor 1820 may be adjusted according to control by the processor 1100.

The output module 1600 is for outputting an audio signal or a video signal. The output module 1600 may include a display 1610 and an audio output module 1620.

When the display 1610 and a touchpad form a layer structure to construct a touch screen, the display 1610 may be used as an input device as well as an output device. The display panel may include at least one of a liquid crystal display (LCD), a thin-film transistor LCD (TFT-LCD), an organic light-emitting diode (OLED), a flexible display, a three-dimensional (3D) display, or an electrophoretic display. Also, the air conditioner 1000 may include two or more displays 1610 according to an implemented configuration thereof.

According to an embodiment of the present disclosure, the display 1610 may display an operating mode of the air conditioner 1000, a current indoor temperature, a current indoor humidity, a current wind speed (or air volume), a desired temperature, etc., but is not limited thereto.

The audio output module 1620 may output audio data received from the communication module 1300 or stored in the memory 1400. For example, the audio output module 1620 may output an audio signal related to a function performed by the air conditioner 1000 (e.g., a notification sound, a guidance voice, or a guide voice).

The output module 1600 may include an output module of a remote control device (remote control). For example, the operating mode of the air conditioner 1000, the desired temperature, the current wind speed, the current indoor temperature, the current indoor humidity, etc. may be displayed through the output module of the remote control device (remote control). In addition, a guidance voice for the user may be output from the remote control device (remote control).

According to an embodiment of the present disclosure, the output module 1600 may also output information related to a sterilization mode. For example, the output module 1600 may output text, an image (e.g., an icon), or a voice indicating that the sterilization mode is to start. For example, the output module 1600 may output text, an image, or a voice asking whether to start the sterilization mode.

The compressor 1200 may compress a refrigerant.

The processor 1100 may generally control all operations of the air conditioner 1000. For example, the processor 1100 may generally control the UV light output module 1830, the output module 1600, the compressor 1200, the fan 1810, the fan motor 1820, the communication module 1300, the input interface 1500, the sensor 1700, a power supply unit (not shown), etc.

According to an embodiment of the present disclosure, the processor 1100 may include a plurality of processors. In addition, the processor 1100 may include, but is not limited to, an artificial intelligence (AI) processor for generating a learning network model.

According to an embodiment of the present disclosure, the processor 1100 may execute one or more instructions stored in the memory 1400 to determine whether a situation of perceived cooling dissatisfaction has occurred.

The communication module 1300 may include one or more components that enable communication between the air conditioner 1000 and a server (not shown), the air conditioner 1000 and a smart device (not shown), or the air conditioner 1000 and an external sensor device (not shown). For example, the communication module 1300 may include, but is not limited to, a short-range communication module.

The short-range communication module may include, but is not limited to, a Bluetooth communication module, a Bluetooth Low Energy (BLE) communication module, a near field communication (NFC) communication module, a wireless local area network (WLAN) (or Wi-Fi) communication module, a ZigBee communication module, an Infrared Data Association (IrDA) communication module, a Wi-Fi Direct (WFD) communication module, an ultra-wideband (UWB) communication module, an Ant+ communication module, etc.

The sensor 1700 may include the illuminance sensor 1710. The illuminance sensor 1710 may detect the intensity of reflected light.

In addition, the sensor 1700 may include, but is not limited to, a humidity sensor (not shown) and a temperature sensor (not shown). The humidity sensor (not shown) may be a sensor for measuring humidity in the air. The temperature sensor (not shown) may be a sensor for measuring the temperature of the air. In addition, functions of the respective sensors may be intuitively inferred by a person having ordinary skill in the art from their names, so detailed descriptions thereof will be omitted.

The input interface 1310 may refer to a device through which the user inputs a signal for controlling the air conditioner 1000. For example, the input interface 1500 may include a keypad, a dome switch, a touch pad (a capacitive overlay type, a resistive overlay type, an infrared beam type, a surface acoustic wave type, an integral strain gauge type, a piezoelectric type, etc.), a jog wheel, a jog switch, etc., but is not limited thereto.

According to an embodiment of the present disclosure, the input interface 1500 may include, but is not limited to, a power button, an operating mode button (e.g., a sterilization mode, a comfort mode, a cooling mode, a dehumidification mode, a cleaning mode, a wind-free function button, a desired temperature setting button, a reservation setting button, a volume adjustment button, a sleep button, an automatic sterilization button, etc.

The input interface 1500 may further include a microphone 1510 for receiving a voice input from the user. The microphone 1312 may receive an external sound signal and process the external sound signal into electrical audio data. For example, the microphone 1510 may receive a sound signal (e.g., a voice command) from an external device or a speaker. The microphone 1510 may use various noise removal algorithms to remove noise that occurs in the process of receiving an external sound signal.

According to an embodiment of the present disclosure, the input interface 1500 may include a remote control device (remote control) and a remote control receiving module 1520. The remote control device (remote control) may include, but is not limited to, a power button, a voice recognition button, an operating mode button, a cleaning function button, a voice recognition microphone, a wind-free function button, a MAX button, a movement and adjustment button, a temperature and air volume adjustment button, an additional function selection button, etc. According to an embodiment of the present disclosure, when a user utters a voice command while pressing a voice recognition button on the remote control device (remote control), the remote control device may recognize the user's voice command.

The processor 1100 may receive, via the input interface 1500, a user input for setting an air volume of wind to be discharged.

The remote control receiving module 1520 may receive a control signal from the remote control device. For example, the remote control receiving module 1520 may receive a control signal input by the user from the remote control device via infrared communication.

The memory 1400 may store programs for processing and controlling the processor 1100, and it may also store input/output data (e.g., operation mode information, user setting information, temperature data, humidity data, notification settings, device information, wind speed information, etc.).

The memory 1400 may include at least one type of storage medium among a flash memory-type memory, a hard disk-type memory, a multimedia card micro-type memory, a card-type memory (e.g., a Secure Digital (SD) card or an eXtreme Digital (XD) memory), random access memory (RAM), static RAM (SRAM), read-only memory (ROM), electrically erasable programmable ROM (EEPROM), PROM, a magnetic memory, a magnetic disc, and an optical disc. Programs stored in the memory 1400 may be categorized into a plurality of modules according to their functions. At least one Al model may be stored in the memory 1400.

The air conditioner 1000 may further include the power supply unit (not shown). The power supply unit (not shown) may supply power to components of the air conditioner 1000 under the control of the processor 1100. The power supply unit (not shown) may supply power input from an external power source via a power cord to each of the components of the air conditioner 1000 under the control of the processor 1100.

The processor 1100 may control the UV light output module 1830 to irradiate UV light onto the fan 1810. When the fan 1810 stops after the fan motor 1820 stops operating, the processor 1100 may determine whether a partial region of the fan 1810 irradiated with UV light is a region sterilized by the UV light output module 1830.

Furthermore, based on determining that the partial region is a sterilized region, the processor 1100 may change a region to be sterilized by driving the fan motor 1820 to rotate the fan 1810.

In addition, based on determining that the partial region is a non-sterilized region, the processor 1100 may sterilize the partial region.

The processor 1100 may receive, via the illuminance sensor 1710, light reflected by a reflective material attached to the fan 1810 via the illuminance sensor 1710. The processor 1100 may determine, based on an intensity of the received reflected light, whether a partial region of the fan 1810 irradiated with UV light is a region sterilized by the UV light output module 1830.

The fan motor 1820 may include a plurality of Hall sensors 1825.

The processor 1100 may determine, based on a sensor value of the Hall sensors 1825, whether a partial region of the fan 1810 irradiated with UV light is a region sterilized by the UV light output module 1830.

The processor 1100 may change a region to be sterilized by applying a driving voltage to the fan motor 1820 during a driving time for changing a sterilization region after the fan 1810 is stopped, and not applying the driving voltage to the fan motor 1820 after the driving time has elapsed.

The processor 1100 may drive the fan motor 1820 based on an RPM lower than a lowest RPM for a cooling operation of the air conditioner 1000 when driving the fan motor 1820 for changing a sterilization region.

Based on determining that the partial region of the fan 1810 is a sterilized region after rotating the fan 1810 by driving the fan motor 1820 at a first RPM, the processor 1100 may rotate the fan 1810 again by driving the fan motor 1820 at an RPM different than the first RPM.

When the fan 1810 stops based on a user input for stopping the cooling operation of the air conditioner 1000, the processor 1100 may sterilize the fan 1810 by driving the UV light output module 1830.

Based on a user input for starting the cooling operation of the air conditioner 1000, the processor 1100 may terminate sterilization of the fan 1810 by stopping operation of the UV light output module 1830.

The processor 1100 may start sterilizing the fan 1810 by driving the UV light output module 1830 based on a user input for starting sterilizing the fan 1810.

A machine-readable storage medium may be provided in the form of a non-transitory storage medium. In this regard, the term 'non-transitory' only means that the storage medium does not include a signal (e.g., an electromagnetic wave) and is a tangible device, and the term does not differentiate between where data is semi-permanently stored in the storage medium and where the data is temporarily stored in the storage medium. For example, the 'non-transitory storage medium' may include a buffer in which data is temporarily stored.

According to an embodiment, methods according to various embodiments of the present disclosure set forth herein may be included in a computer program product when provided. The computer program product may be traded, as a product, between a seller and a buyer. The computer program product may be distributed in the form of a machine-readable storage medium (e.g., compact disc-ROM (CD-ROM)) or distributed (e.g., downloaded or uploaded) on-line via an application store or directly between two user devices (e.g., smartphones). For online distribution, at least a part of the computer program product (e.g., a downloadable app) may be at least transiently stored or temporally generated in the machine-readable storage medium such as memory of a server of a manufacturer, a server of an application store, or a relay server.

## Claims

1. An air conditioner (1000) comprising:
a fan (1810);
a fan motor (1820);
an ultraviolet (UV) light output module (1830);
at least one memory (1400) storing one or more instructions; and
at least one processor (1100), wherein the at least one processor (1100) is configured to execute the one or more instructions stored in the memory (1400) to
sterilize a partial region of the fan (1810) by controlling the UV light output module (1830) to irradiate UV light onto the partial region,
after sterilizing the partial region, change a region to be sterilized by rotating the fan (1810),
when the fan (1810) stops, determine whether a region of the fan (1810) being irradiated with the UV light is a region sterilized by the UV light output module (1830), and
based on determining that the region of the fan (1810) being irradiated with the UV light is a sterilized region, change a region to be sterilized by driving the fan motor (1820) to rotate the fan (1810) again.

2. The air conditioner of claim 1, further comprising
an illuminance sensor,
wherein the at least one processor is further configured to execute the one or more instructions stored in the memory to
receive, via the illuminance sensor, light reflected by a reflective material attached to the fan, and based on an intensity of the received reflected light, determine whether a region of the fan being irradiated with the UV light is a region sterilized by the UV light output module.

3. The air conditioner of claim 2, wherein the reflective material comprises a reflective tape, and blades of the fan respectively have reflective tapes with different reflectivities from each other attached thereto.

4. The air conditioner of any one of claims 1 to 3, wherein
the fan motor comprises a plurality of Hall sensors, and
the at least one processor is further configured to execute the one or more instructions stored in the memory to
determine, based on a sensor value of the Hall sensors, whether a region of the fan being irradiated with the UV light is a region sterilized by the UV light output module.

5. The air conditioner of any one of claims 1 to 4, wherein the at least one processor is further configured to execute the one or more instructions stored in the memory to change the region to be sterilized by applying a driving voltage to the fan motor during a driving time for changing a sterilization region after the fan is stopped, and not applying the driving voltage to the fan motor after the driving time for changing the sterilization region has elapsed.

6. The air conditioner of any one of claims 1 to 5, wherein the at least one processor is further configured to execute the one or more instructions stored in the memory to drive the fan motor based on a revolutions per minute (RPM) lower than a lowest RPM for a cooling operation of the air conditioner.

7. The air conditioner of any one of claims 1 to 6, wherein the at least one processor is further configured to execute the one or more instructions stored in the memory to, based on determining that the partial region of the fan is a sterilized region after rotating the fan by driving the fan motor at a first RPM, rotate the fan again by driving the fan motor at an RPM different than the first RPM.

8. The air conditioner of any one of claims 1 to 7, wherein the at least one processor is further configured to execute the one or more instructions stored in the memory to, when the fan stops based on a user input for stopping the cooling operation of the air conditioner 1000, sterilize the fan by driving the UV light output module.

9. The air conditioner of any one of claims 1 to 8, wherein the at least one processor is further configured to execute the one or more instructions stored in the memory to, based on a user input for starting the cooling operation of the air conditioner, terminate sterilization of the fan by stopping operation of the UV light output module.

10. The air conditioner of any one of claims 1 to 9, wherein the at least one processor is further configured to execute the one or more instructions stored in the memory to, based on determining that a region of the fan being irradiated with the UV light is a sterilized region, change a region to be sterilized by driving the fan motor to rotate the fan again, and based on determining that the region of the fan being irradiated with the UV light is a non-sterilized region, sterilize the region of the fan.

11. A method of sterilizing a fan, the method comprising:
sterilizing a partial region of the fan by irradiating ultraviolet (UV) light onto the partial region;
after sterilizing the partial region, changing a region to be sterilized by rotating the fan;
when the fan stops, determining whether a region of the fan being irradiated with the UV light is a sterilized region; and
based on determining that the region of the fan being irradiated with the UV light is a sterilized region, changing a region to be sterilized by rotating the fan again.

12. The method of claim 11, wherein the determining of whether the region of the fan being irradiated with the UV light is a sterilized region when the fan stops comprises:
receiving light reflected by a reflective material attached to the fan; and
based on an intensity of the received reflected light, determining whether a region of the fan being irradiated with the UV light is a sterilized region.

13. The method of claim 12, wherein
the reflective material comprises a reflective tape, and
blades of the fan respectively have reflective tapes with different reflectivities from each other attached thereto.

14. The method of any one of claims 11 to 13, wherein the determining of whether the region of the fan being irradiated with the UV light is a sterilized region when the fan stops comprises determining, based on a sensor value of a Hall sensor within a fan motor, whether a region of the fan being irradiated with the UV light is a sterilized region.

15. The method of any one of claims 11 to 14, wherein the changing of the region to be sterilized by rotating the fan comprises changing the region to be sterilized by applying a driving voltage to the fan motor during a driving time for changing a sterilization region after the fan is stopped, and not applying the driving voltage to the fan motor after the driving time for changing the sterilization region has elapsed.
